Europäisches Patentamt

European Patent Office

Office européen des brevets

(19)

(11)  **EP 1 128 867 B1**

(12)  ## FASCICULE DE BREVET EUROPEEN

(45) Date de publication et mention
de la délivrance du brevet:
**26.01.2005  Bulletin 2005/04**

(21) Numéro de dépôt: **99954082.6**

(22) Date de dépôt: **08.11.1999**

(51) Int Cl.⁷: **A61N 1/04**, A61N 1/30

(86) Numéro de dépôt international:
**PCT/FR1999/002726**

(87) Numéro de publication internationale:
**WO 2000/027467 (18.05.2000 Gazette 2000/20)**

(54) **ELECTRODE DE TRANSFERT D'UN COURANT ELECTRIQUE TRAVERSANT LA PEAU D'UN PATIENT**

ELEKTRODE ZUM ÜBERTRAGEN VON ELEKTRISCHEM STROM DURCH DIE HAUT EINES PATIENTEN

ELECTRODE FOR TRANSFERRING AN ELECTRIC CURRENT THROUGH A PATIENT'S SKIN

(84) Etats contractants désignés:
**AT BE CH CY DE DK ES FI FR GB GR IE IT LI LU MC NL PT SE**

(30) Priorité:  **09.11.1998  FR 9814072**

(43) Date de publication de la demande:
**05.09.2001  Bulletin 2001/36**

(73) Titulaire: **IOMED, INC.**
**Salt Lake City, Utah 84104 (US)**

(72) Inventeurs:
• **McADAMS, Eric, Thomas**
**County Antrim, N.Ireland, BT38 9PZ (GB)**

• **ZHOU, Dao, Min**
**Castaic, CA 91384 (US)**
• **MAILLEY, Pascal, André, Nicolas**
**F-38000 Grenoble (FR)**
• **MIKLER, Claude**
**F-21000 Dijon (FR)**

(74) Mandataire: **Colas, Jean-Pierre**
**Cabinet JP Colas**
**37, avenue Franklin D. Roosevelt**
**75008 Paris (FR)**

(56) Documents cités:
**WO-A-98/26760          WO-A-98/39057**
**US-A- 4 736 752          US-A- 4 776 350**
**US-A- 5 456 710**

## Description

La présente invention est relative à une électrode de transfert d'un courant électrique traversant la peau d'un patient et, plus particulièrement, à une telle électrode du type qui comprend a) une couche conductrice de l'électricité pour l'amenée ou la collection dudit courant électrique traversant la peau, et b) une couche consommable d'interfaçage électrochimique.

Des électrodes de ce type sont utilisées dans diverses applications médicales, telles que le relevé de signaux électriques originaires du corps humain ou animal (électrocardiogramme, électroencéphalogramme, etc...), l'administration transdermique de médicament par ionophorèse ou l'électrocicatrisation de plaies ou blessures.

Les dispositifs conçus pour mettre en oeuvre ces applications comprennent normalement deux électrodes, l'une faisant fonction d'anode et l'autre de cathode, placées au contact de la peau, le courant appliqué, ou à relever, passant entre ces deux électrodes.

Chaque électrode est couramment formée d'une couche conductrice métallique, par exemple en argent, à laquelle est associée (ou accolée) une couche de conduction ionique constituée d'un gel en contact avec la peau du patient, ce gel étant soit chargé d'un électrolyte assurant un bon contact électrique entre la peau et la couche d'argent (dans le cas du relevé de signaux électriques originaires du corps humain ou animal, par exemple) soit chargé d'un principe actif sous forme ionique (dans le cas de l'administration transdermique de médicaments par ionophorèse).

Dans le cas de l'électrocicatrisation de plaies ou blessures, ladite couche de conduction ionique associée à la couche conductrice est une couche hydrophile en contact cette fois avec la plaie à traiter.

Cette couche hydrophile est soit une couche rendue conductrice initialement, c'est-à-dire avant ou dès son application sur la plaie, par une charge d'eau et de sels minéraux soit une couche hydrophile sèche non conductrice lors de son application sur la plaie et qui devient conductrice suite à la migration des exsudats de la plaie dans l'épaisseur de ladite couche hydrophile sèche.

Dans ce cas, la couche hydrophile sèche qui constitue la couche de conduction ionique peut être réalisée à partir de tout matériau ou composition absorbantes hydrophiles sèches employées dans la réalisation de pansements destinés à traiter les plaies exsudatives.

Elle peut ainsi se présenter sous forme de mousses absorbantes, en particulier de mousses de polyuréthanne hydrophiles employées dans les pansements dits hydrocellulaires, sous forme de fibres à base de matériaux absorbants comme par exemple des fibres d'alginate, tels les alginates de sodium ou de calcium, ou des fibres de dérivés cellulosiques, sous forme de compresses en non-tissé, sous forme de gels lyophilisés et sous forme de compositions hydrocolloïdes telles des gels ou des masses hydrocolloïdes telles celles employées dans les pansements de type dit hydrocolloïdes.

Dans tous les cas, la conduction électrique passe d'une conduction électronique (dans la couche d'argent) à une conduction ionique (dans le gel). Le transfert de charge permettant cet échange est contrôlé par les propriétés électrochimiques de l'interface électrode/peau.

Pour assurer la stabilité de ces propriétés électrochimiques, on a proposé de former sur la couche d'argent précitée une couche d'interfaçage électrochimique propre à faciliter le passage de la conduction ionique à la conduction électrochimique en maintenant, à l'interface en question, un potentiel électrochimique faible et stable, assurant un transfert de charges de fort rendement.

Cette couche d'interfaçage, dite aussi couche "sacrificielle", est classiquement constituée d'argent ou d'un mélange Ag/AgCl, dans le cas d'une anode ou d'une cathode, respectivement, déposée sur ou mélangée à la couche d'amenée de courant. L'interfaçage électrochimique propre à ces couches sacrificielles est alors assuré par la réaction d'oxydo-réduction réversible :

$$Ag + Cl^- \Leftrightarrow AgCl + e^-$$

qui transforme par oxydation l'argent de l'anode en chlorure d'argent et par réduction le chlorure d'argent de la cathode en argent. Le potentiel de la réaction est relativement faible (de 100 à 200 mV environ) par rapport au potentiel de l'électrode standard à hydrogène. La différence de potentiel entre anode et cathode est faible et du même ordre. En outre, le potentiel standard du couple Ag/AgCl se situant dans la fenêtre de stabilité électrochimique de l'eau, cette réaction empêche tout phénomène d'électrolyse de l'eau accompagné de variations de pH et de formation de gaz, ce qui assure la stabilité électrochimique de l'interface et la sécurité du patient quant à l'une de ces nuisances liées à la dérive de l'interface électrochimique, et ceci aussi longtemps que ladite réaction peut avoir lieu, c'est-à-dire aussi longtemps que du chlorure d'argent est présent à la cathode et/ou que de l'argent et l'ion Cl⁻ sont disponibles à l'anode.

Bien que de telles couches d'interfaçage répondent ainsi à la demande en ce qui concerne la stabilité électrochimique de l'interface électrode/peau, on s'est aperçu, lorsque le traitement exige le passage de forts courants pendant de longues périodes de temps, comme c'est le cas dans les dispositifs ionophorétiques d'administration transdermique de médicaments, que les électrodes équipées de telles couches d'interfaçage laissaient encore à désirer en ce qui concerne leur résistance à une dégradation physique, l'uniformité de la densité du courant sur la surface de l'électrode et la "capacité électrochimique" de celle-ci, c'est-à-dire son aptitude à délivrer un courant d'intensité donné (de l'ordre de 1 µA à 1 mA, plus précisément de l'ordre de 0,2 mA par cm$^2$ de surface d'électrode dans le cas d'une application à l'ionophorèse,) pendant un intervalle de temps donné (de plusieurs heures ou plus) comme cela est nécessaire pour que l'on puisse assurer un contrôle précis d'une administration transdermique de médicament par ionophorèse.

On observe en effet, dans cette application, une certaine dislocation de l'électrode, due notamment à un défaut d'adhérence de la couche d'Ag/AgCl sur la couche d'argent sous-jacente. On observe également une non uniformité de la densité du courant traversant des plages adjacentes de l'électrode, cette densité tendant à s'accroître sur les bords de l'électrode. Ce défaut d'uniformité crée des "points chauds" sur ces bords qui irritent la peau du patient. En outre, elle provoque une érosion plus rapide de la couche d'interfaçage électrochimique au droit des points chauds de l'électrode, qui voit alors sa durée de vie baisser. Cet abaissement entraîne celui de la capacité électrochimique de l'électrode.

Le document WO-A-9 839 957 concerne un dispositif pour la délivrance d'un médicament par ionophorèse avec une couche conductrice et une couche d'interfaçage électrochimique.

EP-A-0 985 426, publiée après la date de dépôt de la demande présente (document appartenant à l'état de la technique telle que défini à l'art.54(3) CBE), décrit une électrode comprenant une couche conductrice de le'électricité, une couche intermédiaire en un matériau inerte chimiquement et électriquement résistant et une couche consommable d'interfaçage électrochimique.

La présente invention a précisément pour but de fournir une électrode du type décrit ci-dessus, perfectionnée de manière à présenter une stabilité mécanique et une capacité électrochimique supérieure à celle des électrodes de la technique antérieure, c'est-à-dire une durée de vie compatible avec la durée des traitements thérapeutiques qui font usage de telles électrodes, tels que l'administration transdermique de médicaments par ionophorèse, ou encore l'électrocicatrisation.

On atteint ces buts de l'invention, ainsi que d'autres qui apparaîtront à la lecture de la description qui va suivre, avec une électrode de transfert d'un courant électrique traversant la peau d'un patient selon la rev. 1.

Comme on le verra plus loin en détail, la présence de cette couche intermédiaire améliore à la fois la tenue mécanique de l'électrode et sa capacité électrochimique.

Suivant un mode de réalisation préféré de l'invention, la couche intermédiaire comprend du carbone, par exemple sous la forme de fines particules dispersées dans un liant polymère.

Suivant un autre mode de réalisation préféré de l'invention, l'électrode est formée sur un substrat en matériau électrochimiquement isolant sur lequel est accolée la couche conductrice de l'électricité.

L'électrode comprend en outre une couche de conduction ionique en contact avec une plaie ou la peau d'un patient.

Suivant une caractéristique optionnelle de l'électrode selon l'invention, celle-ci comprend en outre une couche d'un matériau électriquement isolant, recouvrant les bords de la pile de couches constituée par la couche conductrice, la couche intermédiaire et la couche d'interfaçage électrochimique.

Suivant encore une autre caractéristique optionnelle de l'électrode selon l'invention, la couche conductrice prend la forme d'une grille.

D'autres caractéristiques et avantages de la présente invention apparaîtront à la lecture de la description qui va suivre et à l'examen du dessin annexé dans lequel :

-    la figure 1 est une vue en coupe schématique d'une électrode suivant l'invention, appliquée sur la peau d'un patient,
-    la figure 2 est une vue éclatée de l'électrode de la figure 1,
-    la figure 3 est une vue schématique d'un mode de réalisation d'une couche d'amenée de courant formant partie de l'électrode des figures 1 et 2, et
-    la figure 4 représente des graphes qui illustrent l'accroissement de la capacité électrochimique de l'électrode suivant l'invention, par rapport à celle d'une électrode de la technique antérieure.

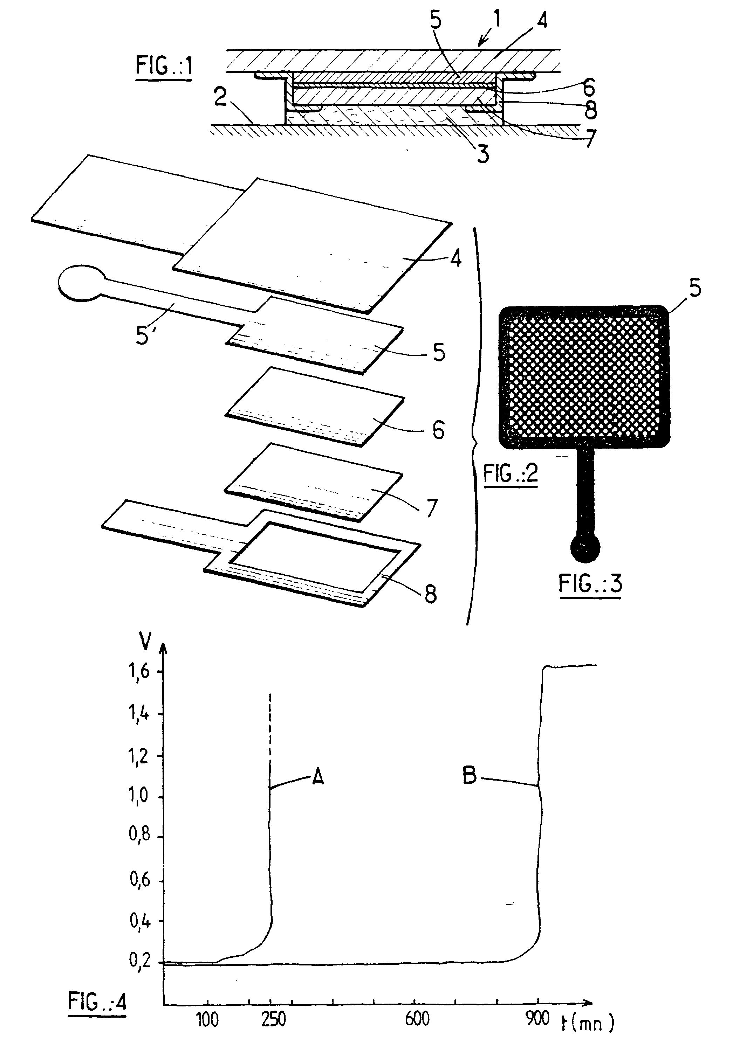

On se réfère à la figure 1 du dessin annexé où l'on a représenté schématiquement un mode de réalisation de l'électrode 1 suivant l'invention, dans la position qu'elle occupe sur la peau 2 d'un patient. Sur cette figure 1, on a fortement dilaté l'électrode dans son épaisseur pour rendre plus visible les diverses couches de matériaux qui la constituent. Dans la suite, on va décrire l'électrode en tant qu'élément d'un dispositif ionophorétique d'administration transdermique de médicaments, à titre d'exemple illustratif et non limitatif seulement, une telle électrode pouvant être utilisée dans d'autres dispositifs, comme on le verra dans la suite.

Un dispositif ionophorétique d'administration transdermique de médicaments comprend classiquement un circuit électronique conçu pour débiter un courant d'intensité prédéterminée pendant un intervalle de temps prédéterminé, entre deux électrodes appliquées sur la peau d'un patient. L'une des électrodes est elle-même accolée à un "réservoir" tel celui référencé 3 à la figure 1, en contact direct avec la peau dudit patient, ce réservoir contenant en solution une forme ionique d'un principe actif constituant le médicament à administrer. Suivant la polarité de cette forme ionique, l'électrode en question est une anode ou une cathode, le champ électrique établi par la circulation du courant entre les deux électrodes forçant les ions du principe actif à passer du réservoir 3, constitué par une couche de conduction ionique formée d'un gel hydrophile chargé d'une solution saline dudit principe actif, par exemple, sous la partie adjacente de la peau dudit patient.

L'autre électrode peut elle-même être aussi accolée à une couche de gel hydrophile chargé d'une solution saline, pour le seul but d'assurer une bonne conduction électrique entre la peau et l'électrode, nécessaire au passage du courant entre celles-ci.

Les deux couches de gel peuvent être intégrées chacune à une des électrodes et "hydratées" avec les solutions décrites ci-dessus, juste avant la mise en route d'un traitement.

On revient aux figures 1 et 2 du dessin annexé où il apparaît que l'électrode suivant l'invention comprend plusieurs couches de différents matériaux, accolées les unes aux autres. C'est ainsi que l'électrode peut comprendre d'abord une couche 4 d'un substrat, avantageusement souple, enduit sur une face d'une couche 5 en un matériau conducteur de l'électricité. La souplesse du substrat permet d'appliquer étroitement l'électrode sur la peau du patient.

Suivant la présente invention, la couche 5 est elle-même recouverte d'une couche 6 en un matériau électriquement résistant, inerte chimiquement, intermédiaire entre la couche conductrice 5 et une couche consommable d'interfaçage électrochimique 7, du type décrit ci-dessus en préambule de la présente description.

La pile des couches 5,6,7 décrites ci-dessus, peut être recouverte, sur ses bords, par une couche 8 d'un matériau diélectrique dessinant un cadre pour un but que l'on expliquera plus loin.

L'électrode 1 ainsi constituée peut être complétée par le dépôt, sur les couches 7 et 8, de la couche de gel 3 évoquée ci-dessus.

Diverses techniques bien connues de réalisation de films minces ou de couches de matériaux peuvent être utilisées pour déposer les couches précitées, dépôt sous vide en phase vapeur, enduction au rouleau, dépôt sérigraphique, etc... Cette dernière technique est préférée pour la réalisation de l'électrode des figures 1 et 2.

De ce qui précède il résulte que l'électrode suivant l'invention se distingue essentiellement des électrodes connues de la technique antérieure par la présence de la couche 6 en un matériau électriquement résistant et inerte chimiquement.

Suivant un mode de réalisation de l'invention, cette couche 6 est une couche à base de carbone résistive et fine, de quelques micromètres d'épaisseur, constituée, par exemple, par une dispersion de fines particules de carbone dans un liant polymère. Ces particules peuvent présenter une taille de 0,1 à 5 µm, la teneur en carbone de l'encre étant de l'ordre de 15 à 25 % en poids du mélange.

Suivant un autre mode de réalisation de l'invention, la couche 6 est à résistivité anisotropique. Un matériau convenable pour constituer une telle couche est, par exemple, celui vendu sous le nom "z-axis adhesive film" par la société des USA dite Minnesota Mining and Manufacturing Co.

La couche 6 est interposée entre la couche 5, en argent par exemple, et la couche d'interfaçage 7 en Ag/AgCl pour la cathode, en argent pour l'anode, cette couche 7 étant elle-même en contact direct avec la couche 3 de gel hydrophile chargé d'une solution ionique conductrice de l'électricité.

La couche 5 assure la liaison électrique avec le circuit électronique qui commande le passage d'un courant électrique entre de telles électrodes. La couche 7 assure la stabilité des propriétés électrochimiques de l'interface de l'électrode avec la couche de gel et la peau du patient.

La couche intermédiaire 6, composée par exemple de carbone, de l'électrode suivant l'invention joue d'abord, grâce à son inertie chimique, le rôle d'une "couche tampon" qui assure :

1) que l'adhérence entre les couches 5 et 7 n'est pas affectée par le passage du courant et l'épuisement de la couche "sacrificielle" d'Ag/AgCl, dans le cas d'une cathode ou d'argent dans le cas d'une anode,
2) que la conductivité de la couche 5 n'est pas affectée par les réactions électrochimiques qui se développent dans la couche d'interfaçage électrique 7, et
3) que l'adhérence de la couche 5 sur le substrat 4 n'est pas détériorée par ces réactions électrochimiques.

Suivant la présente invention, la couche intermédiaire 6 doit aussi présenter une résistance électrique plus élevée que celle de la couche conductrice 5 sous-jacente. Des mesures ont montré en effet qu'il en résulte une distribution du courant plus uniforme sur la surface de la couche sacrificielle 7, qui atténue "l'effet de bord" noté en préambule de la présente description en l'absence de la couche en carbone. Il en résulte une "consommation" plus régulière de la couche sacrificielle 7, au fur et à mesure du passage d'un courant, ce qui accroît alors la durée de vie de l'électrode. Celle-ci est alors capable de faire circuler sous la peau d'un patient un courant d'une intensité donnée, pendant un temps plus long, qu'une électrode dépourvue de la couche 6 en carbone, ce qui accroît la "capacité électrochimique" de l'électrode suivant l'invention par rapport à celle d'une électrode dépourvue de cette couche 6.

Il apparaît ainsi que la présente invention permet bien d'atteindre les buts annoncés en préambule de la présente description, à savoir réaliser une électrode présentant une stabilité mécanique et électrochimique d'une part, une capacité électrochimique d'autre part, améliorées.

On renforce encore les résultats énoncés ci-dessus grâce à la couche 8 isolante qui recouvre notamment les bords des couches 5, 6 et 7 sous-jacentes. Incidemment on remarquera que cette couche sert aussi à isoler, en la recouvrant, une piste d'alimentation 5' de la couche 5, débordant du contour de cette couche.

La couche 8 protège les couches 5 et 6 de la couche de gel 3 et empêche ainsi qu'un solvant contenu dans cette couche ne puisse pénétrer, par les bords, jusque dans la couche 5, ce qui minimise les risques de "délamination" des couches 5 et 6.

Cette couche 8, électriquement isolante, s'oppose aussi à la présence de plages à forte densité de courant sur les bords de l'électrode, créatrice de "points chauds" à la fois désagréables pour le patient par l'irritation de la peau qu'elle peut engendrer, et nuisible à la capacité électrochimique de l'électrode par l'érosion précoce de la couche sacrificielle qu'elle provoque.

On va maintenant décrire un mode de réalisation de l'électrode suivant l'invention, à titre d'exemple illustratif et non limitatif seulement.

EXEMPLE

La couche de substrat 4 utilisée pour supporter les autres couches est constituée par un film de polyester de 50 micromètres d'épaisseur, environ.

Les couches 5,6,7 sont déposées successivement sur le substrat 4 par la technique bien connue de la sérigraphie.

La couche 5 peut être constituée par toute "encre" conductrice, typiquement une encre à base d'argent, susceptible de présenter une bonne adhérence à la fois sur le substrat 4 et sur la couche intermédiaire 6 qui la recouvre. La couche 5 peut présenter un réseau de lacunes ou "trous", comme représenté à la figure 3 de manière à prendre la forme d'une grille. Ces lacunes permettent à la couche 6 d'adhérer directement au substrat à travers elles, ce qui améliore la stabilité mécanique de l'électrode et crée une multitude de courbures dans les lignes du courant amené, qui assurent une meilleure répartition dudit courant du fait de la multiplication des effets de bord. Les déformations que cette grille imprime aux couches 6 et 7 qui la recouvrent accroissent la surface de l'interface couche 7/gel 3, ou de l'interface couche 7/électrolyte favorable aux propriétés électriques de cette interface.

La couche intermédiaire 6 doit être résistive et mince, ou de résistitivé anisotropique, et inerte chimiquement. On peut utiliser pour la constituer une encre conductrice du commerce à base de fines particules en carbone dispersées dans un liant polymère et un solvant. De tels encres sont commercialisées par les sociétés DuPont de Nemours, Acheson Colloiden BV ou encore Gwent Electronic Materials, par exemple. D'autres matériaux résistifs pourraient être utilisés pour constituer la couche 6 : encre à base de graphite telle celle commercialisée sous le nom BIROX par la société DuPont de Nemours, encres à base d'oxydes de ruthénium, de palladium, de thallium ou d'iridium, encre à base de palladium ou encore d'un alliage palladium-argent. L'encre doit présenter les propriétés d'adhérence requises et permettre de séparer physiquement la couche sacrificielle 7 de la couche conductrice 5 d'argent. Quand la couche 7 est en Ag/AgCl, la couche 6 assure que l'adhérence de cette dernière couche sur les couches sous-jacentes n'est pas affectée par le passage du courant et l'épuisement qui en résulte du AgCl de la couche 7, que les propriétés de conduction électrique de la couche 5 ne sont pas affectées par les réactions électrochimiques qui se développent de l'autre côté de la couche 6, et que le substrat 4 n'est pas lui non plus atteint par lesdites réactions. De préférence, la couche intermédiaire présente une épaisseur comprise entre 1 et 100 micromètres et préférablement entre 5 et 10 µm, dans le cas d'une couche résistive mince.

La couche sacrificielle 7 est constituée classiquement, comme on l'a vu plus haut, d'une encre à base d'argent, pour l'anode, d'une encre à base d'Ag/AgCl pour la cathode. Pour une même encre, l'épaisseur et la rugosité de cette couche de cathode détermine pour l'essentiel la capacité électrochimique telle que définie ci-dessus, c'est-à-dire la durée de vie utile de celle-ci. Pour donner à cette couche l'épaisseur convenable pour atteindre la durée de vie souhaitée, on pourra la réaliser avec un écran à larges mailles ou bien en superposant plusieurs couches déposées successivement.

La couche diélectrique 8 est réalisée avec une encre isolante, par exemple une encre pour film épais durcissable sous UV référencée 451ss dans les catalogues de la société hollandaise ACHESON COLLOIDEN BV.

On a réalisé des électrodes selon les données énoncées ci-dessus. Les couches 5,6,7,8 présentaient des épaisseurs comprises entre 5 et 20 µm, 5 et 10 µm, 20 et 70 µm, et 5 à 20 µm respectivement. Préférablement, les couches 5,6,7,8 présentaient respectivement des épaisseurs de 12 µm, 8 µm, 40 µm et 8 à 10 µm. La composition de la couche 7 pour une cathode était comprise entre 40 et 80 % en poids de AgCl et 60 à 20 % d'argent et plus préférablement, entre 30 et 40 % d'Ag et 60 à 70 % d'AgCl.

Les graphes de la figures 4 illustrent l'accroissement de la capacité électrochimique obtenue par la présente invention. Ils présentent l'évolution dans le temps de la tension relevée entre la couche 5 et un électrolyte baignant la couche 7 ou la couche de gel 3, lorsqu'on fait passer dans l'électrode un courant de 1 mA. Les graphes A et B représentent l'évolution de cette tension pour une électrode dépourvue de la couche 6 en carbone et pour une électrode suivant l'invention, respectivement. Dans les deux cas, on commence par relever une tension de l'ordre de 0,2 V correspondant au faible potentiel électrochimique de la réaction d'oxydoréduction réversible engendrée par la présence de la couche sacrificielle 7. Au bout d'un certain temps, celle-ci s'épuise et le potentiel électrochimique passe à une valeur très supérieure, de l'ordre de 1,6 V, soit au potentiel électrochimique de la réaction d'électrolyse de l'eau, solvant de l'électrolyte. A ce moment, l'électrode est dégradée et cesse d'être utilisable.

On relève couramment un temps de 250/300 minutes pour l'apparition de cette tension, avec une électrode dépourvue de la couche 6 en carbone (graphe A) et une densité de courant de 125 $\mu A/cm^2$.

La présence d'une couche 6 constituée d'une encre DuPont référencée E 84380-106A fait passer ce temps à 900 minutes environ (graphe B) au moins, avec la même densité de courant, et ceci sans que l'on observe les fissures et décollages de couches mentionnés ci-dessus.

La "capacité électrochimique " Q de l'électrode suivant l'invention s'en trouve accrue d'autant, pour passer d'une valeur centrée sur 40 mA.mn/cm$^2$ environ, à une valeur centrée sur 187 $\mu$A.mn/cm$^2$ et d'au moins 168 $\mu$A.mn/cm$^2$, la surface de l'électrode étant mesurée en cm$^2$, conformément à l'un des objectifs poursuivis par la présente invention.

Bien entendu, l'invention n'est pas limitée au mode de réalisation décrit et représenté qui n'a été donné qu'à titre d'exemple. C'est ainsi que les diverses couches de l'électrode suivant l'invention pourraient être réalisées par des procédés d'enduction autres que la sérigraphie. Ainsi, on peut trouver dans le commerce des substrats chargés en carbone donnant à ces substrats une certaine résistance électrique qui leur permet de jouer le rôle de la couche intermédiaire 6. On forme alors sur une face de ce substrat une couche métallique, par dépôt en phase vapeur par exemple, pour constituer la couche 5 et, sur l'autre face du substrat, la couche d'interfaçage électrochimique 7.

De même, l'invention n'est pas limitée à la réalisation d'électrodes pour dispositifs ionophorétiques d'administration transdermique de médicaments. Elle trouve au contraire application partout où un équilibre électrochimique doit être maintenu à l'interface où s'applique l'électrode, et notamment donc à des électrodes utilisées pour l'électrothérapie des plaies ou blessures, des électrodes de détection des signaux électriques originaires du corps humain (électroencéphalogrammes, électrocardiogrammes) et à des électrodes d'électrostimulation musculaire transdermique (TENS).

**Revendications**

1. Electrode de transfert d'un courant électrique traversant la peau d'un patient, **caractérisée en ce qu'**elle comprend, dans cet ordre :

   a) une couche (5) conductrice de l'électricité pour l'amenée ou la collection dudit courant électrique traversant ladite peau,
   b) une couche intermédiaire (6) en un matériau inerte chimiquement et électriquement résistant,
   c) une couche (7) consommable d'interfaçage électrochimique, et
   d) une couche de conduction ionique (3) destinée à venir en contact avec la peau du patient.

2. Electrode conforme à la revendication 1, **caractérisée en ce que** la couche intermédiaire (6) comprend du carbone.

3. Electrode conforme à la revendication 2, **caractérisée en ce que** la couche intermédiaire (6) est constituée de fines particules de carbone dispersées dans un liant polymère.

4. Electrode conforme à l'une quelconque des revendications 1 à 3, **caractérisée en ce que** ladite couche (6) intermédiaire présente une épaisseur comprise entre 1 $\mu$m et 100 $\mu$m environ, préférablement entre 5 et 10 $\mu$m.

5. Electrode conforme à l'une quelconque des revendications précédentes, **caractérisée en ce que** ladite couche de conduction ionique (3) est destinée à venir en contact avec une plaie et est constituée par une couche hydrophile sèche non conductrice de l'électricité au moment de son application sur la plaie et qui devient conductrice suite à la migration des exsudats de la plaie dans l'épaisseur de ladite couche hydrophile sèche.

6. Electrode conforme à l'une quelconque des revendications 1 à 4, **caractérisée, en ce que** ladite couche de conduction ionique (3) est destinée à venir en contact avec une plaie et est une couche hydrophile qui peut être rendue conductrice avant son application sur la plaie par une charge d'eau et de sels minéraux.

7. Electrode conforme à l'une quelconque des revendications précédentes, **caractérisée en ce qu'**elle comprend en outre une couche (8) d'un matériau électriquement isolant, recouvrant les bords de la pile de couches constituée par la couche conductrice (5), la couche intermédiaire (6) et la couche d'interfaçage électrochimique (7).

8. Electrode conforme à l'une quelconque des revendications précédentes, **caractérisée en ce que** la couche conductrice (5) prend la forme d'une grille.

9. Electrode conforme à l'une quelconque des revendications précédentes, **caractérisée en ce qu'**elle est formée sur un substrat (4) en un matériau électriquement isolant sur lequel est accolée la couche (5) conductrice de l'électricité.

**10.** Electrode conforme à l'une quelconque des revendications 1 à 8, **caractérisée en ce que** la couche intermédiaire (6) est constituée par un matériau de substrat chargé de particules de carbone.

**11.** Electrode conforme à l'une quelconque des revendications précédentes, **caractérisée en ce que** ladite couche intermédiaire présente une résistivité anisotropique.

**12.** Electrode conforme à l'une quelconque des revendications précédentes, **caractérisée en ce que** la couche conductrice (5) comprend de l'argent.

**13.** Electrode conforme à l'une quelconque des revendications précédentes, **caractérisée en ce que** la couche d'interfaçage électrochimique (7) comprend de l'argent.

**14.** Electrode conforme à l'une quelconque des revendications 1 à 11, **caractérisée en ce que** la couche d'interfaçage électrochimique (7) comprend un mélange Ag/AgCl.

**15.** Electrode conforme à la revendication 14, **caractérisée en ce que** ledit mélange comprend entre 40 et 80 % en poids de AgCl et 60 à 20 % d'argent, préférablement entre 30 et 40 % d'Ag et 60 à 70 % d'AgCl.

**16.** Electrode conforme à l'une quelconque des revendications précédentes, **caractérisée en ce qu'**elle présente une capacité électrochimique d'au moins 168 $\mu$A.mn.cm$^2$.

**Claims**

**1.** Electrode for the transfer of an electrical current flowing through the skin of a patient, **characterized in that** it comprises, in this order:

a) an electrically conductive layer (5) for feeding or collecting said electrical current flowing through said skin,
b) an intermediate layer (6) of a chemically inert and electrically resistive material,
c) a consumable electrochemical interface layer (7), and
d) an ionic conduction layer (3) adapted to come into contact with the skin of the patient.

**2.** Electrode according to claim 1, **characterized in that** the intermediate layer (6) contains carbon.

**3.** Electrode according to claim 2, **characterized in that** the intermediate layer (6) consists of fine particles of carbon dispersed in a polymer binder.

**4.** Electrode according to any one of claims 1 to 3, **characterized in that** said intermediate layer (6) is from approximately 1 $\mu$m to approximately 100 $\mu$m thick and preferably from 5 $\mu$m to 10 $\mu$m thick.

**5.** Electrode according to any one of the preceding claims, **characterized in that** said ionic conduction layer (3) is adapted to come into contact with a wound and consists of a dry hydrophilic layer that is not electrically conductive when it is applied to the wound and becomes conductive following the migration of exudate from the wound into the thickness of said dry hydrophilic layer.

**6.** Electrode according to any one of claims 1 to 4, **characterized in that** said ionic conduction layer (3) is adapted to come into contact with a wound and is a hydrophilic layer that may be rendered conductive before its application to the wound by a charge of water and mineral salts.

**7.** Electrode according to any one of the preceding claims, **characterized in that** it further comprises a layer (8) of an electrically insulative material covering the edges of the stack of layers consisting of the conductive layer (5), the intermediate layer (6), and the electrochemical interface layer (7).

**8.** Electrode according to any one of the preceding claims, **characterized in that** the conductive layer (5) takes the form of a grid.

**9.** Electrode according to any one of the preceding claims, **characterized in that** it is formed on a substrate (4) of an electrically insulative material in contact with the electrically conductive layer (5).

**10.** Electrode according to any one of claims 1 to 8, **characterized in that** the intermediate layer (6) consists of a substrate material charged with particles of carbon.

**11.** Electrode according to any one of the preceding claims, **characterized in that** said intermediate layer has an anisotropic resistivity.

**12.** Electrode according to any one of the preceding claims, **characterized in that** the conductive layer (5) contains silver.

**13.** Electrode according to any one of the preceding claims, **characterized in that** the electrochemical interface layer (7) contains silver.

**14.** Electrode according to any one of claims 1 to 11, **characterized in that** the electrochemical interface layer (7) contains an Ag/AgCl mixture.

**15.** Electrode according to claim 14, **characterized in that** said mixture comprises from 40 to 80% by weight of AgCl and from 60 to 20% of silver, preferably from 30 to 40% of Ag and from 60 to 70% of AgCl.

**16.** Electrode according to any one of the preceding claims, **characterized in that** it has an electrochemical capacity of at least 168 $\mu$A.mn.cm$^2$.

**Patentansprüche**

**1.** Elektrode zum Übertragen eines elektrischen Stroms durch die Haut eines Patienten, **dadurch gekennzeichnet, dass** sie in der folgenden Reihenfolge Folgendes umfasst:

a) eine Strom leitende Schicht (5) zum Zuführen oder Sammeln des elektrischen Stroms, der die Haut durchquert,

b) eine Zwischenschicht (6) aus einem chemisch trägen und elektrisch widerstehenden Werkstoff,

c) eine Verbrauchsschicht (7) zur elektrochemischen Schnittstellenbildung und

d) eine ionisch leitende Schicht (3), die dazu bestimmt ist, mit der Haut des Patienten in Berührung zu kommen.

**2.** Elektrode nach Anspruch 1, **dadurch gekennzeichnet, dass** die Zwischenschicht (6) Kohlenstoff umfasst.

**3.** Elektrode nach Anspruch 2, **dadurch gekennzeichnet, dass** die Zwischenschicht (6) aus feinen Kohlenstoffpartikeln besteht, die in einem Polymerbindemittel dispergiert sind.

**4.** Elektrode nach einem der Ansprüche 1 bis 3, **dadurch gekennzeichnet, dass** die Zwischenschicht (6) eine Stärke zwischen etwa 1 $\mu$m und 100 $\mu$m, vorzugsweise zwischen 5 und 10 $\mu$m aufweist.

**5.** Elektrode nach einem der vorhergehenden Ansprüche, **dadurch gekennzeichnet, dass** die ionisch leitende Schicht (3) dazu bestimmt ist, mit einer Wunde in Berührung zu kommen, und aus einer trockenen, im Augenblick ihrer Anlegung auf die Wunde nicht Strom leitenden hydrophilen Schicht besteht, und die im Anschluss an die Migration der Exsudate der Wunde in die Stärke der hydrophilen trockenen Schicht leitend wird.

**6.** Elektrode nach einem der Ansprüche 1 bis 4, **da**durch gekennzeichnet, dass die leitende ionische Schicht (3) dazu bestimmt ist, mit einer Wunde in Berührung zu kommen und eine hydrophile Schicht ist, die vor ihrem Aufbringen auf die Wunde durch eine Ladung Wasser- und Mineralsalzladung elektrisch leitend gemacht werden kann.

**7.** Elektrode nach einem der vorhergehenden Ansprüche, **dadurch gekennzeichnet, dass** sie ferner eine Schicht (8) aus einem elektrisch isolierenden Werkstoff umfasst, die die Ränder des Stapels der Schichten bedeckt, die aus der leitenden Schicht (5), der Zwischenschicht (6) und der elektrochemischen schnittstellenbildenden Schicht (7) besteht.

**8.** Elektrode nach einem der vorhergehenden Ansprüche, **dadurch gekennzeichnet, dass** die leitende Schicht (5) die Form eines Gitters annimmt.

**9.** Elektrode nach einem der vorhergehenden Ansprüche, **dadurch gekennzeichnet, dass** sie auf einem Substrat (4) aus einem elektrisch isolierenden Werkstoff gebildet wird, an den die elektrisch leitende Schicht (5) angefügt wird.

**10.** Elektrode nach einem der Ansprüche 1 bis 8, **dadurch gekennzeichnet, dass** die Zwischenschicht (6) aus einem Substratwerkstoff besteht, der mit Kohlenstoffpartikeln geladen ist.

**11.** Elektrode nach einem der vorhergehenden Ansprüche, **dadurch gekennzeichnet, dass** die Zwischenschicht eine anisotrope Resistivität aufweist.

**12.** Elektrode nach einem der vorhergehenden Ansprüche, **dadurch gekennzeichnet, dass** die leitende Schicht (5) Silber enthält.

**13.** Elektrode nach einem der vorhergehenden Ansprüche, **dadurch gekennzeichnet, dass** die elektrochemische schnittstellenbildende Schicht (7) Silber enthält.

**14.** Elektrode nach einem der Ansprüche 1 bis 11, **dadurch gekennzeichnet, dass** die elektrochemische schnittstellenbildende Schicht (7) ein Gemisch aus Ag/AgCl enthält.

**15.** Elektrode nach Anspruch 14, **dadurch gekennzeichnet, dass** das Gemisch zwischen 40 und 80 Gew.-% AgCl und 60 bis 20 Gew.-% Silber, vorzugsweise zwischen 30 und 40 Gew.-% Ag und 60 bis 70 Gew.-% AgCl enthält.

**16.** Elektrode nach einem der vorhergehenden Ansprüche, **dadurch gekennzeichnet, dass** sie eine elektrochemische Leistung von mindestens 168 $\mu$A.mn. cm$^2$ aufweist.

FIG.:1

FIG.:2

FIG.:3

FIG.:4